# EUROPEAN PATENT SPECIFICATION

(11) **EP 4 178 514 B1**
(45) Date of publication and mention of the grant of the patent: **07.01.2026**
(21) Application number: 21739796.7
(22) Date of filing: 01.07.2021
(51) Int. Cl.: A61F 15/00, A61F 15/02

(54) **REUSABLE CUTTING ARTICLES AND WOUND TAPE ARTICLES AND KITS CONTAINING THE SAME**
WIEDERVERWENDBARE SCHNEIDARTIKEL UND WUNDBANDARTIKEL UND KITS DAMIT
ARTICLES DE COUPE RÉUTILISABLES AINSI QU'ARTICLES DE BANDE ENROULÉE ET KITS LES CONTENANT

(30) Priority: 09.07.2020 US 202063049717 P
(43) Date of publication of application: 17.05.2023
(73) Proprietor: 3M Innovative Properties Company, Saint Paul, Minnesota 55133-3427 (US)
(72) Inventor: NASH, James E., Saint Paul, Minnesota 55133-3427 (US)
(74) Representative: Vossius & Partner Patentanwälte Rechtsanwälte mbB
(86) International application number: PCT/IB2021/055930
(87) International publication number: WO 2022/009040

(56) References cited:
- US-A1- 2009 179 061
- US-A1- 2019 209 401

## Description

### BACKGROUND

Rolled tape packaged in boxes, such as Scotch^{®} Magic^{™} tape is known. Such rolled tapes can be placed in dispensers. Rolled tapes that sold in single-use disposable dispensers are also known. US 2019/0209401 A1 relates to a bandage dispensing assembly. US 2009/0179061 A1 relates to adhesive tape dispensers made of molded plastic.

### BRIEF DESCRIPTION OF THE DRAWINGS

Figure 1 is three-quarters top-down view of an exemplary removable cutting article;
Figure 2 is a side view of the exemplary removable cutting article as also shown in Figure 1;
Figure 3 is a top view of the exemplary removable cutting article as also shown in Figures 1-2;
Figure 4 is a bottom view of the exemplary removable cutting article as also shown in Figures 1-3;
Figure 5 is a front view of the exemplary removable cutting article as also shown in Figures 1-4;
Figure 6 is a back view of the exemplary removable cutting article as also shown in Figures 1-5;
Figure 7 is a three-quarters top-down view of another exemplary removable cutting article;
Figure 8 is aside view of the exemplary removable cutting article as also shown in Figure 7;
Figure 9 is a top view of the exemplary removable cutting article as also shown in Figures 7-8;
Figure 10 is a bottom view of the exemplary removable cutting article as also shown in Figures 7-9;
Figure 11 is a front view of the exemplary removable cutting article as also shown in Figures 7-10;
Figure 12 is a back view of the exemplary removable cutting article as also shown in Figures 7-11;
Figure 13 is a three-quarters top-down view of yet another exemplary removable cutting article;
Figure 14 is a side view of the exemplary removable cutting article as also shown in Figure 13;
Figure 15 is a top view of the exemplary removable cutting article as also shown in Figures 13-14;
Figure 16 is a bottom view of the exemplary removable cutting article as also shown in Figures 31-15;
Figure 17 is a front view of the exemplary removable cutting article as also shown in Figures 13-16;
Figure 18 is a back view of the exemplary removable cutting article as also shown in Figures 31-17;
Figure 19 is a view of the removable cutting article of Figures 1-6 affixed to a box;
Figure 20 is a view of the removable cutting article of Figures 7-12 affixed to a box;
Figure 21 is a view of the removable cutting article of Figures 13-18 affixed to a box;
Figure 22 is a view of a wound tape article featuring the removable cutting article of Figures 7-12; and
Figure 23 is a view of a wound tape article featuring the removable cutting article of Figures 7-12.

### DETAILED DESCRIPTION

Throughout this disclosure, singular forms such as "a," "an," and "the" are often used for convenience; however, the singular forms are meant to include the plural unless the singular alone is explicitly specified or is clearly indicated by the context. When the singular alone is called for, the term "one and only one" is typically used.

Some terms in this disclosure are defined below. Other terms will be familiar to the person of skill in the art and should be afforded the meaning that a person of ordinary skill in the art would have ascribed to them.

Terms indicating a high frequency, such as (but not limited to) "common," "typical," and "usual," as well as "commonly," "typically," and "usually" are used herein to refer to features that are often employed in the invention and, unless specifically used with reference to the prior art, are not intended to mean that the features are present in the prior art, much less that those features are common, usual, or typical in the prior art.

Current wound tape products are often sold in disposable dispensers that are designed to be disposed of after a single roll of tape is consumed. These are very convenient because there is no need to load the tape roll into a dispenser, but they generate a large amount of waste. Typically, the disposable dispenser is plastic. Even if it were possible to recycle the plastic dispenser, the use of plastic can be environmentally problematic as it has been reported that only 9% of plastic in the United States is recycled. Compostable plastic dispensers are not known.

Other current wound tape products are sold in boxes to be placed in reusable dispensers. The boxes are often made of recyclable or compostable paper or cardboard, however they are less convenient to use than wound tape products that are sold in a disposable dispenser because they need to be loaded into a reusable dispenser before they can be conveniently used.

This disclosure provides a solution to the foregoing problems and other problems. Briefly, reusable cutting articles, as well as wound tape articles and kits containing the reusable cutting article, that addresse the disadvantages of both formerly known wound tape products. A removable cutting article is configured to releasably engage with a box, such as a box containing a wound tape article, that has a box top, a box front and opposing box back, and two opposite box sides. The invention is specified by the independent claims. Preferred embodiments are defined in the dependent claims. In the following description, although numerous features may be designated as optional, it is nevertheless acknowledged that all features comprised in the independent claims are not to be read as optional.

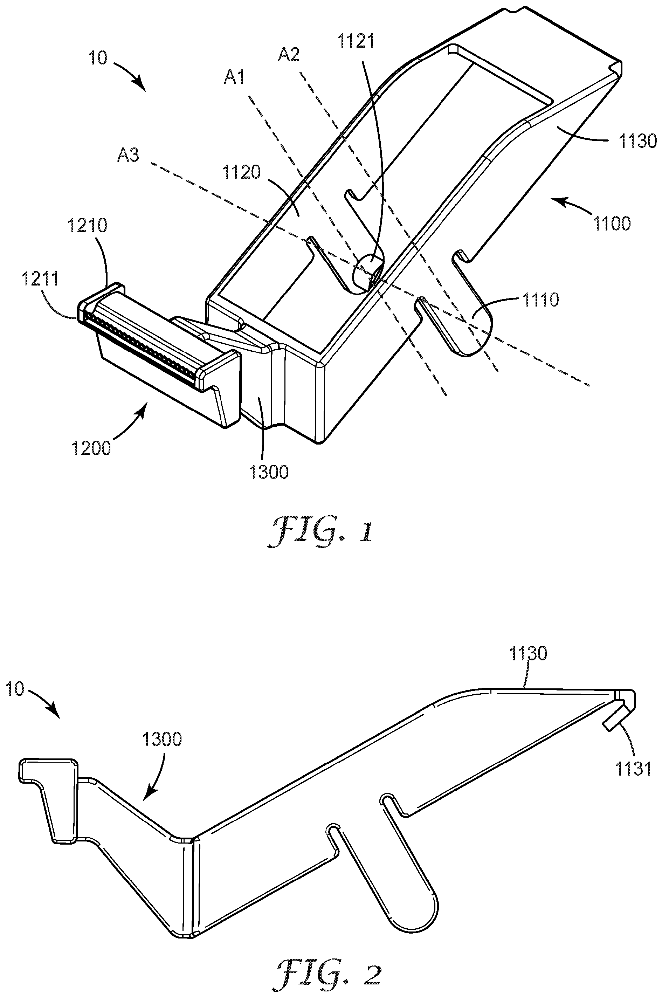

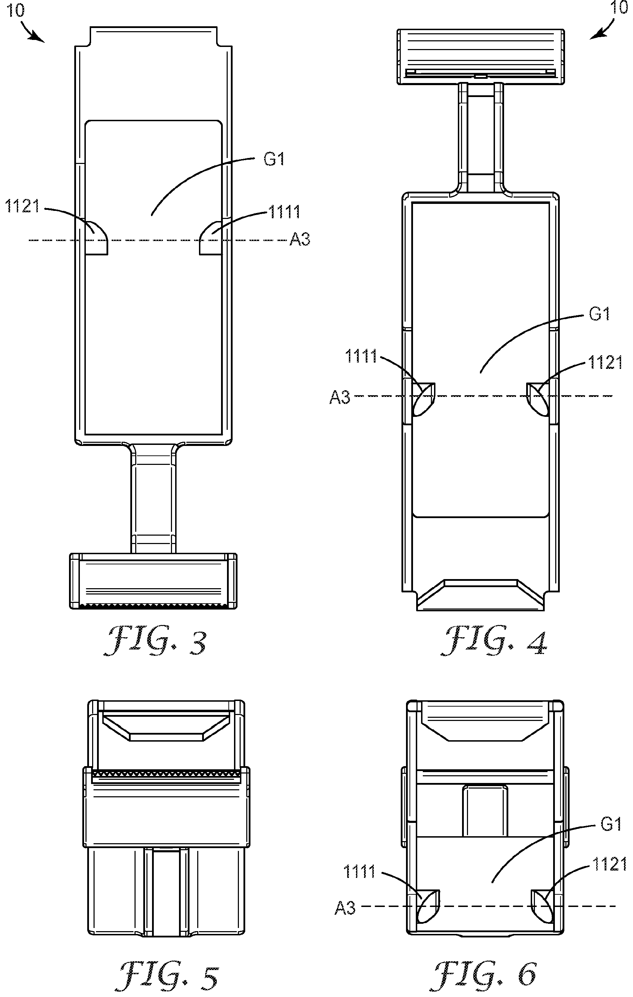

Figures 1-6 show removable cutting article **10** comprising distal end **1100** and proximal end **1200** spaced apart by spacing member **1300.** Distal end **1100** includes two arms **1110, 1120** extending along a first axis **A1** and parallel second axis **A2,** each of which has a protrusion **1111, 1121** extending into gap **G1** that separates arms **1100, 1200** along third axis **A3.** Third axis **A3** is substantially orthogonal to first axis **A1** and second axis **A2.** Distal end **1100** also includes housing **1130,** which in turn comprises clip **1131.** Proximal end **1200** includes cutting element **1210** which is spaced from the two arms **1100, 1200** by spacing member **1300** and comprises sharp edge **1211.**

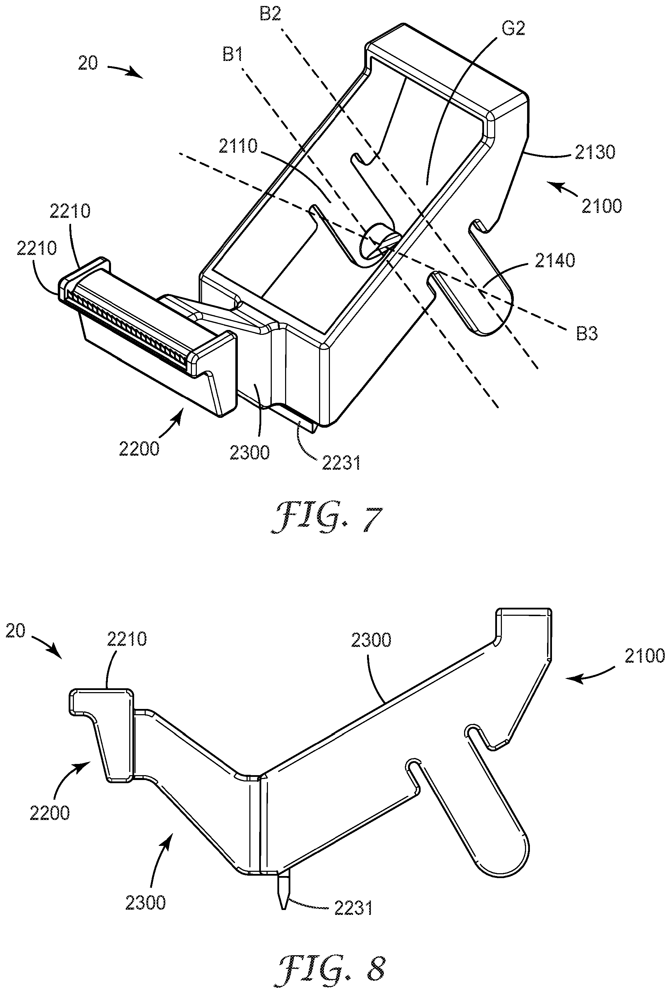

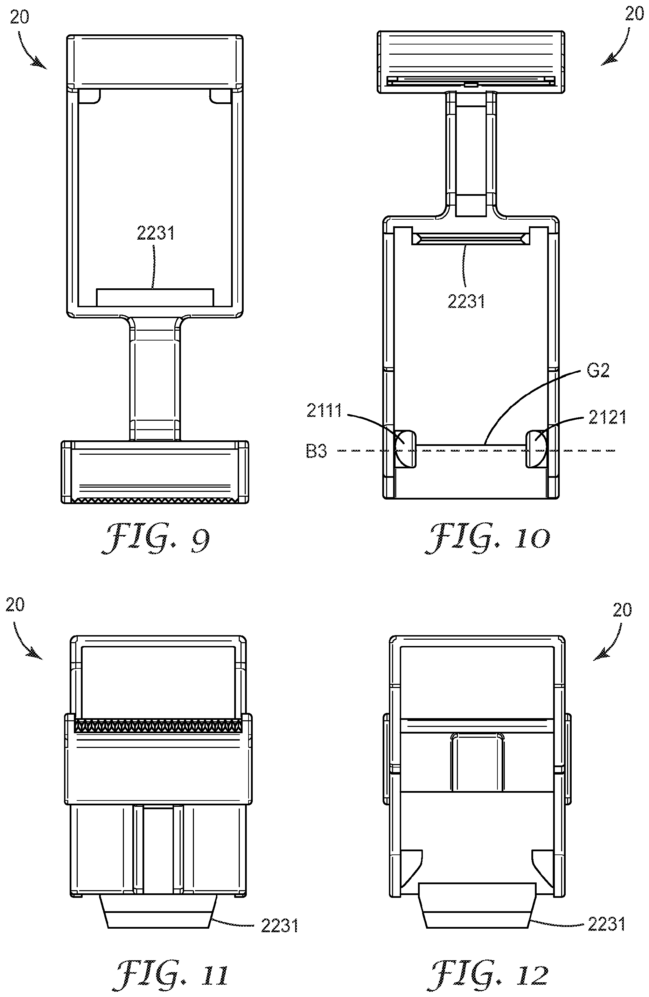

Figures 7-12 show removable cutting article **20** comprising a distal end **2100** and proximal end 2200 spaced apart by spacing member **2300.** Distal end **2100** includes two arms **2110, 2120.** Distal end **2100** includes two arms **2110, 2120** extending along a first axis **B1** and parallel second axis **B2,** each of which has a protrusion **2111, 2121** extending into gap **G2** that separates arms **2100, 2200** along third axis **B3.** Third axis **B3** is substantially orthogonal to first axis **B1** and second axis **B2.** Distal end **2100** also comprises housing **2130.** Proximal end **2200** includes cutting element **2210** which is spaced from the two arms **2100, 2200** by spacing member **2300** and comprises sharp edge **2211.** Clip **2231** is also present on proximal end **2200**

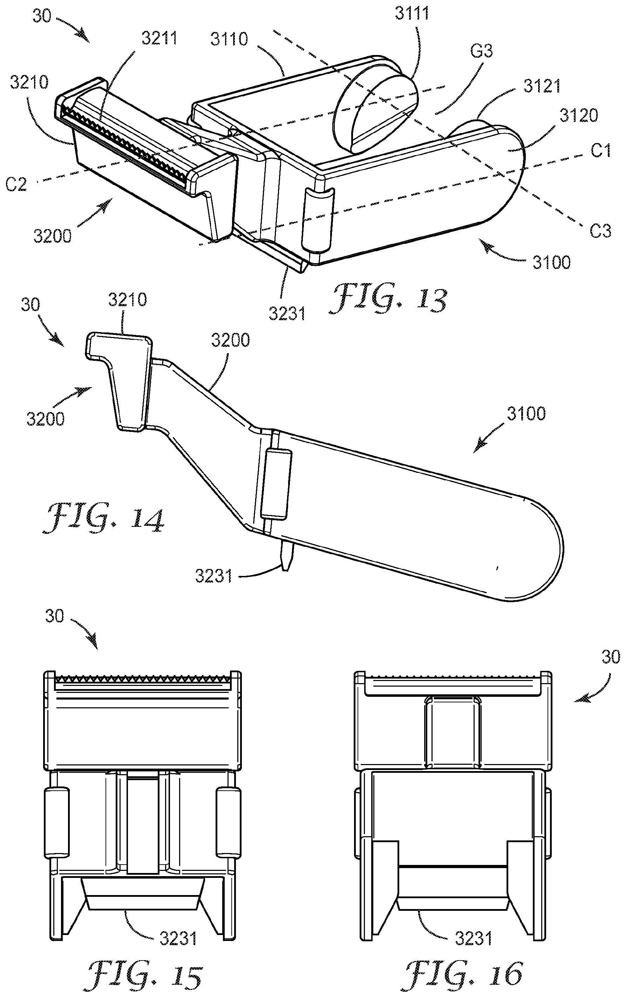

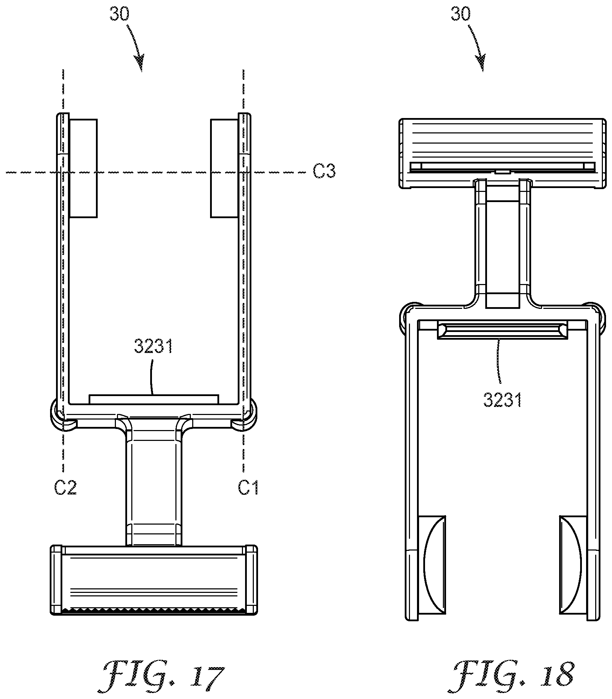

Figures 13-18 show removable cutting article **30** comprising a distal end **3100** and proximal end **3200** spaced apart by spacing member **3300.** Distal end **3100** includes two arms **3110, 3120** extending along a first axis **C1** and parallel second axis **C2,** each of which has a protrusion **3111, 3121** extending into gap **G3** that separates arms **3100, 3200** along third axis **C3.** Third axis **C3** is substantially orthogonal to first axis **B1** and second axis **B2.** Removable cutting article **30** does not have a housing component to distal end **3100.** Proximal end **3200** includes cutting element **3210** which is spaced from the two arms **3100, 3200** by spacing member **3300** and comprises sharp edge **3211.** Clip **3231** is also present on proximal end **3200**

A wound tape article can be formed by placing a removable cutting article on a box that contains tape. Any removable cutting article disclosed herein can be used for this purpose. The box containing the tape can have one or more openings for attaching the removable cutting article. The one or more openings can be made in any suitable way or shape. For example, openings preformed in the box, or there can be perforations or scores in where the openings are to be formed by a user when the removable cutting article is placed on the box.

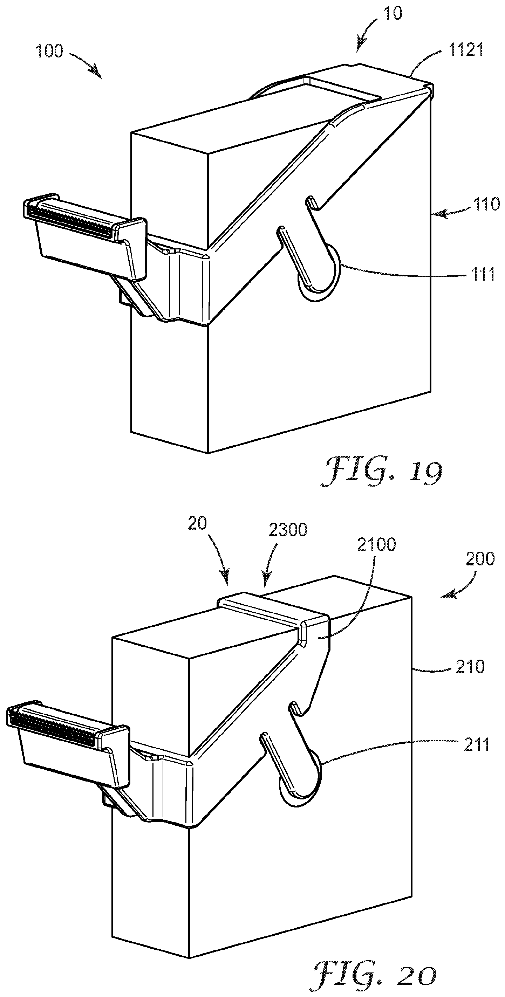

Figure 19 shows removable cutting article **10,** which is depicted in more detail in Figures 1-6, as a component of wound tape article **100,** that also includes box **110** having opening **111** for receiving protrusions **1111, 1121** (not visible in this Figure). Clip **1131** helps secure removable cutting article **10** on box **110,** either by gripping the outside of box **110** or by punching out a perforation (not shown) in an appropriate location on box **110.** Not visible in this Figure is a wound tape roll within box **110.**

Figure 20 shows removable cutting article **20,** which is depicted in more detail in Figures 7-12, as a component of wound tape article **200,** that also includes box **210** having opening **211** for receiving protrusions **2111, 2121** (not visible in this Figure). Housing **2300** of distal end **2100** is configured to help retain removable cutting element **20** on box **210.** Clip **2231** (not visible in this figure) helps secure removable cutting article **20** to box **210** by punching out a perforation (not shown) in an appropriate location on box **210.**

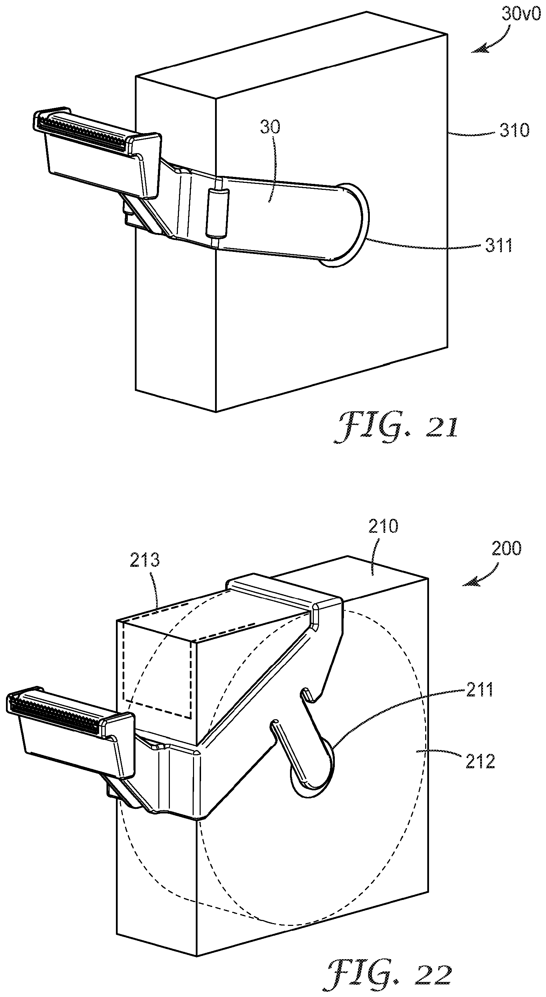

Figure 21 shows removable cutting article **30,** which is depicted in more detail in Figures 13-18, as a component of wound tape article **300,** that also includes box **310** and opening **311** for receiving protrusions **3111, 3121** (not visible in this Figure). Proximal end **3200** of removable cutting article **30** is configured to keep removable cutting article **30** in place on box **310.** Clip **3231** (not visible in this figure) helps secure removable cutting article **30** to box **310** by punching out a perforation (not shown) in an appropriate location on box **310.** Alternatively, clip **3231** might be inserted into a pre-existing slot (rather than using perforations).

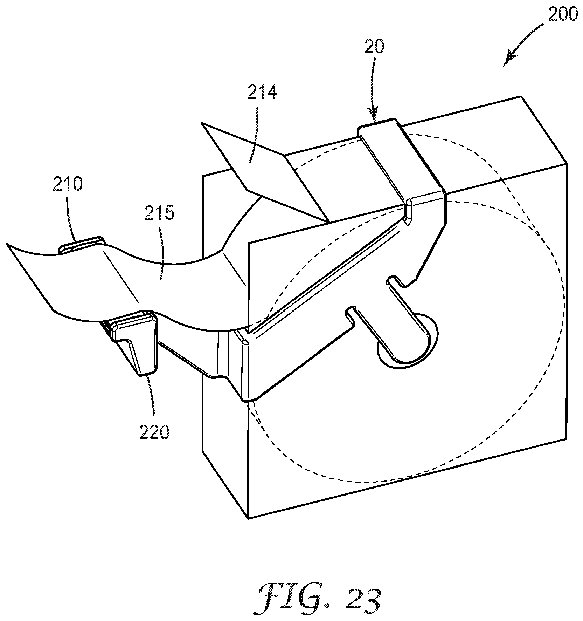

As an example of how the removable cutting articles and wound tape articles herein may be used, Figures 22 and 23 depict wound tape article **200** having tape roll **212** located such that the center of tape roll **212** is aligned with opening **211.** A core (not visible in this Figure) may optionally be employed to retain tape roll **212** centered on protrusions **3111, 3121** (not visible in this Figure) as tape is removed from tape roll **212.** Perforations **213** (Figure 22) allow access to tape roll **212.**

When perforations **213** are torn, flap **214** allows access to tape **215,** which can be unrolled to the desired length over proximal end **2200,** and more specifically cutting element **2210,** of removable cutting article **20.** This allows the wound tape article **200** to be used in essentially the same manner as a standard tape dispenser.

Any type of tape that can be wound can be employed, including tapes with single-sided or double-sided adhesive, foam tapes, cellulose based tapes, masking tapes, conspicuity tapes, magnetic tape, and the like. The tapes may have, for example, a circular core or a flat core (flat-wound tape), or they may have no core. One exemplary tape that can be used is the tape available as SCOTCH^{®} Brand MAGIC^{™} TAPE (3M Company, St. Paul, MN, USA); other types of tape can also be employed.

A removable cutting article, such as those described herein, can be included as part of a kit with one or more, such as one to 6, rolls or boxes of tape. The removable cutting article can be attached to a roll or box of tape as shown in the Figures to make wound tape articles.

## Claims

1. A removable cutting article (10; 20; 30) for releasably engaging with a box (110; 210; 310) that has a box top, a box front and opposing box back, and two opposite box sides, the removable cutting article (10; 20; 30) comprising:
a distal end (1100; 2100; 3100) and a proximal end (1200; 2200; 3200) connected by a spacing member (1300; 2300; 3300);
the distal end (1100; 2100; 3100) comprising two arms (1110, 1120; 2110, 2120; 3110, 3120) that are spaced apart from each other and that are oriented along a first axis (A1; B1; C1) and a second axis (A2; B2; C2) parallel to the first axis (A1; B1; C1),
wherein each of the two arms has a protrusion (1111, 1121; 2111, 2121; 3111, 3121) that extends from the arms and towards each other along a third axis (A3; B3; C3) that is substantially orthogonal to the first axis (A1; B1; C1);
the proximal end (1200; 2200; 3200) comprises at least one cutting element (1210; 2210; 3210) spaced from the at least two arms (1110, 1120; 2110, 2120; 3110, 3120), the cutting element having a sharp edge (1211; 2211; 3211) that is disposed along a fourth axis that is substantially parallel to the third axis (A3; B3; C3).

2. The removable cutting article of claim 1, wherein the two arms (1110, 1120; 2110, 2120; 3110, 3120) are separated by a gap (G1; G2; G3) and further wherein the protrusion of each of the two arms extends into the gap (G1; G2; G3).

3. The removable cutting article of any of the preceding claims, wherein the distal end (1100; 2100) further comprises a housing (1130; 2130) that is connected to the two arms (1110, 1120; 2110, 2120).

4. The removable cutting article of any of the preceding claims, wherein the housing (1130; 2130) comprises space for engaging with the box (110; 210; 310).

5. The removable cutting article of any of the preceding claims, wherein the housing (1130; 2130) comprises a back for engaging with the box top or the box back.

6. A wound tape article (100; 200; 300) comprising the box (110; 210; 310) according to any one of the preceding claims containing a wound tape roll and the removable cutting article (10; 20; 30) according to any one of the preceding claims removably attached to the box (110; 210; 310).

7. The wound tape article of claim 6, wherein the box sides each comprise a hole for receiving the at least a portion of the protrusions (1111, 1121: 2111, 2121; 3111, 3121) of the removable cutting article (10; 20; 30), and wherein at least a portion of one of the protrusions are disposed within each hole.

8. The wound tape article of any of claims 6 or 7, wherein the box (110; 210; 310) comprises a slot in the box top for receiving a portion of the housing (1130; 2130) and wherein at least a portion of the housing is disposed within the slot.

9. The wound tape article of any of claims 6-8, wherein the box (110; 210; 310) comprises a slot in the box back for receiving a portion of the housing (1130; 2130) and wherein at least a portion of the housing is disposed within the slot.

10. The wound tape article of any of claims 6-9, wherein the box (110; 210; 310) comprises an opening in the box front or the box top for removing the tape and wherein the sharp edge (1211; 2211; 3211) is spaced from the box front and is located between the opening and the box bottom.

11. The wound tape article of any of claims 6-10, further comprising a core around which the tape is wound, the core optionally comprising a hollow center, within the box, and
wherein the tape is wound around the core.

12. The wound tape article of any of claims 7-11, wherein the holes are aligned with a core within the box, wherein the tape is wound around the core.

13. The wound tape article of any of claims 6-12, wherein the box comprises perforations (213).

14. A kit comprising a plurality of boxes that contain one wound roll of tape; and
a removable cutting article (10; 20; 30) of any of claims 1-5.

15. The kit of claim 14, comprising from one to twenty, optionally from one to ten, and optionally from one to six boxes that contain one wound roll of tape.

## Patentansprüche

1. Ein entfernbarer Schneidgegenstand (10; 20; 30) zum lösbaren Eingreifen in eine Schachtel (110; 210; 310), die eine Schachteloberseite, eine Schachtelvorderseite und eine gegenüberliegende Schachtelrückseite und zwei gegenüberliegende Schachtelseiten aufweist, der entfernbare Schneidgegenstand (10; 20; 30) aufweisend:
ein distales Ende (1100; 2100; 3100) und ein proximales Ende (1200; 2200; 3200), die durch ein Abstandselement (1300; 2300; 3300) verbunden sind;
das distale Ende (1100; 2100; 3100) aufweisend zwei Arme (1110, 1120; 2110, 2120; 3110, 3120), die voneinander beabstandet sind und die entlang einer ersten Achse (A1; B1; C1) und einer zweiten Achse (A2; B2; C2) parallel zu der ersten Achse (A1; B1; C1) orientiert sind,
wobei jeder der zwei Arme einen Vorsprung (1111, 1121; 2111, 2121; 3111, 3121) aufweist, der sich von den Armen und zueinander entlang einer dritten Achse (A3; B3; C3) erstreckt, die im Wesentlichen orthogonal zu der ersten Achse (A1; B1; C1) ist;
das proximale Ende (1200; 2200; 3200) aufweisend mindestens ein Schneidelement (1210; 2210; 3210), das von den mindestens zwei Armen (1110, 1120; 2110, 2120; 3110, 3120) beabstandet ist, wobei das Schneidelement eine scharfe Kante (1211; 2211; 3211) aufweist, die entlang einer vierten Achse angeordnet ist, die im Wesentlichen parallel zu der dritten Achse (A3; B3; C3) ist.

2. Der entfernbare Schneidgegenstand nach Anspruch 1, wobei die zwei Arme (1110, 1120; 2110, 2120; 3110, 3120) durch einen Spalt (G1; G2; G3) getrennt sind und wobei sich ferner der Vorsprung jedes der zwei Arme in den Spalt (G1; G2; G3) erstreckt.

3. Der entfernbare Schneidgegenstand nach einem der vorstehenden Ansprüche, wobei das distale Ende (1100; 2100) ferner ein Gehäuse (1130; 2130) aufweist, das mit den zwei Armen (1110, 1120; 2110, 2120) verbunden ist.

4. Der entfernbare Schneidgegenstand nach einem der vorstehenden Ansprüche, wobei das Gehäuse (1130; 2130) Platz zum Eingreifen in die Schachtel (110; 210; 310) aufweist.

5. Der entfernbare Schneidgegenstand nach einem der vorstehenden Ansprüche, wobei das Gehäuse (1130; 2130) eine Rückseite zum Eingreifen in die Schachteloberseite oder die Schachtelrückseite aufweist.

6. Ein aufgewickelter Bandgegenstand (100; 200; 300), aufweisend die Schachtel (110; 210; 310) nach einem der vorstehenden Ansprüche, die eine aufgewickelte Bandrolle enthält, und den entfernbaren Schneidgegenstand (10; 20; 30) nach einem der vorstehenden Ansprüche, der an der Schachtel (110; 210; 310) entfernbar befestigt ist.

7. Der aufgewickelte Bandgegenstand nach Anspruch 6, wobei die Schachtelseiten jeweils ein Loch zum Aufnehmen von mindestens einem Abschnitt der Vorsprünge (1111, 1121; 2111, 2121; 3111, 3121) des entfernbaren Schneidgegenstands (10; 20; 30) aufweisen und wobei mindestens ein Abschnitt eines der Vorsprünge innerhalb jedes Lochs angeordnet ist.

8. Der aufgewickelte Bandgegenstand nach einem der Ansprüche 6 oder 7, wobei die Schachtel (110; 210; 310) einen Schlitz in der Schachteloberseite zum Aufnehmen eines Abschnitts des Gehäuses (1130; 2130) aufweist und wobei mindestens ein Abschnitt des Gehäuses innerhalb des Schlitzes angeordnet ist.

9. Der aufgewickelte Bandgegenstand nach einem der Ansprüche 6 bis 8, wobei die Schachtel (110; 210; 310) einen Schlitz in der Schachtelrückseite zum Aufnehmen eines Abschnitts des Gehäuses (1130; 2130) aufweist und wobei mindestens ein Abschnitt des Gehäuses innerhalb des Schlitzes angeordnet ist.

10. Der aufgewickelte Bandgegenstand nach einem der Ansprüche 6 bis 9, wobei die Schachtel (110; 210; 310) eine Öffnung in der Schachtelvorderseite oder der Schachteloberseite zum Entfernen des Bandes aufweist und wobei die scharfe Kante (1211; 2211; 3211) von der Schachtelvorderseite beabstandet ist und sich zwischen der Öffnung und der Schachtelunterseite befindet.

11. Der aufgewickelte Bandgegenstand nach einem der Ansprüche 6 bis 10, ferner aufweisend einen Kern, um den das Band gewickelt ist, der Kern optional aufweisend eine hohle Mitte, innerhalb der Schachtel, und wobei das Band um den Kern gewickelt ist.

12. Der aufgewickelte Bandgegenstand nach einem der Ansprüche 7 bis 11, wobei die Löcher auf einen Kern innerhalb der Schachtel ausgerichtet sind, wobei das Band um den Kern gewickelt ist.

13. Der aufgewickelte Bandgegenstand nach einem der Ansprüche 6 bis 12, wobei die Schachtel Perforationen (213) aufweist.

14. Ein Kit, aufweisend eine Mehrzahl von Schachteln, die eine aufgewickelte Rolle von Band enthalten; und einen entfernbaren Schneidgegenstand (10; 20; 30) nach einem der Ansprüche 1 bis 5.

15. Das Kit nach Anspruch 14, aufweisend von einer bis zwanzig, optional einer bis zehn und optional einer bis sechs Schachteln, die eine aufgewickelte Rolle von Band enthalten.

## Revendications

1. Article de coupe amovible (10 ; 20 ; 30) destiné à mettre en prise de manière libérable une boîte (110 ; 210 ; 310) qui a un dessus de boîte, un avant de boîte et un dos de boîte opposé, et deux côtés de boîte opposés, l'article de coupe amovible (10 ; 20 ; 30) comprenant :
une extrémité distale (1100 ; 2100 ; 3100) et extrémité proximale (1200 ; 2200 ; 3200) reliées par un membre d'espacement (1300 ; 2300 ; 3300) ;
l'extrémité distale (1100 ; 2100 ; 3100) comprenant deux bras (1110, 1120 ; 2110, 2120 ; 3110, 3120) qui sont espacés l'un de l'autre et qui sont orientés le long d'un premier axe (A1 ; B1 ; C1) et d'un deuxième axe (A2 ; B2 ; C2) parallèle au premier axe (A1 ; B1 ; C1),
dans lequel chacun des deux bras ayant une
saillie (1111, 1121 ; 2111, 2121 ; 3111, 3121) qui s'étend à partir des bras et l'un vers l'autre le long d'un troisième axe (A3 ; B3 ; C3) qui est sensiblement orthogonal au premier axe (A1 ; B1 ; C1) ;
l'extrémité proximale (1200 ; 2200 ; 3200) comprenant au moins un élément de coupe (1210 ; 2210 ; 3210) espacés des au moins deux bras (1110, 1120 ; 2110, 2120 ; 3110, 3120), l'élément de coupe ayant un bord tranchant (1211 ; 2211 ; 3211) qui est disposé le long d'un quatrième axe sensiblement parallèle au troisième axe (A3 ; B3 ; C3).

2. Article de coupe amovible selon la revendication 1, dans lequel les deux bras (1110, 1120 ; 2110, 2120 ; 3110, 3120) sont séparés par un écartement (G1 ; G2 ; G3) et en outre dans lequel la saillie de chacun des deux bras s'étend dans l'écartement (G1 ; G2 ; G3).

3. Article de coupe amovible selon l'une quelconque des revendications précédentes, dans lequel l'extrémité distale (1100 ; 2100) comprend en outre un logement (1130 ; 2130) qui est relié aux deux bras (1110, 1120 ; 2110, 2120).

4. Article de coupe amovible selon l'une quelconque des revendications précédentes, dans lequel le logement (1130 ; 2130) comprend un espace pour mettre en prise la boîte (110 ; 210 ; 310).

5. Article de coupe amovible selon l'une quelconque des revendications précédentes, dans lequel le logement (1130 ; 2130) comprend un dos destiné à mettre en prise le dessus de boîte ou le dos de boîte.

6. Article de ruban enroulé (100 ; 200 ; 300) comprenant la boîte (110 ; 210 ; 310) selon l'une quelconque des revendications précédentes contenant un rouleau de ruban enroulé et l'article de coupe amovible (10 ; 20 ; 30) selon l'une quelconque des revendications précédentes attachés de façon amovible à la boîte (110 ; 210 ; 310).

7. Article de ruban enroulé selon la revendication 6, dans lequel les côtés de boîte comprennent chacun un trou destiné à recevoir l'au moins une partie des saillies (1111, 1121 ; 2111 , 2121 ; 3111 , 3121) de l'article de coupe amovible (10 ; 20 ; 30), et dans lequel au moins une partie de l'une des saillies est disposée au sein de chaque trou.

8. Article de ruban enroulé selon l'une quelconque des revendications 6 ou 7, dans lequel la boîte (110 ; 210 ; 310) comprend une fente dans le dessus de boîte destinée à recevoir une partie du logement (1130 ; 2130) et dans lequel au moins une partie du logement est disposée au sein de la fente.

9. Article de ruban enroulé selon l'une quelconque des revendications 6 à 8, dans lequel la boîte (110 ; 210 ; 310) comprend une fente dans le dos de boîte destinée à recevoir une partie du logement (1130 ; 2130) et dans lequel au moins une partie du logement est disposée au sein de la fente.

10. Article de ruban enroulé selon l'une quelconque des revendications 6 à 9, dans lequel la boîte (110 ; 210 ; 310) comprend une ouverture sur le devant de boîte ou le dessus de boîte destinée à retirer le ruban et dans lequel le bord tranchant (1211 ; 2211 ; 3211) est espacé de l'avant de boîte et se situe entre l'ouverture et le fond de boîte.

11. Article de ruban enroulé selon l'une quelconque des revendications 6 à 10, comprenant en outre un noyau autour duquel le ruban est enroulé, le noyau comprenant éventuellement un centre creux, au sein de la boîte, et dans lequel le ruban est enroulé autour du noyau.

12. Article de ruban enroulé selon l'une quelconque des revendications 7 à 11, dans lequel les trous sont alignés avec un noyau au sein de la boîte, dans lequel le ruban est enroulé autour du noyau.

13. Article de ruban enroulé selon l'une quelconque des revendications 6 à 12, dans lequel la boîte comprend des perforations (213).

14. Kit comprenant une pluralité de boîtes qui contiennent un rouleau de ruban enroulé ; et un article de coupe amovible (10 ; 20 ; 30) selon l'une quelconque des revendications 1 à 5.

15. Kit selon la revendication 14, comprenant de une à vingt, éventuellement de une à dix, et éventuellement de une à six boîtes qui contiennent un rouleau de ruban enroulé.
